(19) Europäisches Patentamt
European Patent Office
Office européen des brevets

(11) **EP 2 157 790 A1**

## (12) EUROPEAN PATENT APPLICATION
published in accordance with Art. 153(4) EPC

(43) Date of publication:
**24.02.2010 Bulletin 2010/08**

(21) Application number: **07767366.3**

(22) Date of filing: **21.06.2007**

(51) Int Cl.:
*H04N 5/93* (2006.01)     *A61B 1/00* (2006.01)
*H04N 5/66* (2006.01)     *H04N 5/915* (2006.01)
*H04N 7/18* (2006.01)

(86) International application number:
**PCT/JP2007/062540**

(87) International publication number:
**WO 2008/155861 (24.12.2008 Gazette 2008/52)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Olympus Corporation**
**Tokyo 151-0072 (JP)**

(72) Inventor: **KANDA, Yamato**
**Tokyo 151-0072 (JP)**

(74) Representative: **Gunzelmann, Rainer**
**Wuesthoff & Wuesthoff**
**Patent- und Rechtsanwälte**
**Schweigerstraße 2**
**D-81541 München (DE)**

## (54) IMAGE DISPLAY DEVICE AND IMAGE DISPLAY PROGRAM

(57)     An image obtaining unit 710 reads, after time-series images taken by a capsule endoscope are stored in a portable recording medium 50, the time-series images from the portable recording medium 50, thereby obtaining the time-series images. An image-display-condition setting unit 761 receives an input that specifies display conditions including a minimum display rate R_min, which corresponds to a longest display time for which one image is displayed, via an input unit 720 and sets the display conditions by the received user operation. An importance-coefficient calculating unit 751 calculates an importance coefficient that represents the degree of importance of each image. A display-time calculating unit 753 creates display-time data 745 by calculating a display time for each image. An image-display control unit 763 performs control by referring to the display-time data 745 so that the images are displayed sequentially in the temporal order for the respective display times.

FIG.3

**Description**

TECHNICAL FIELD

The present invention relates to an image display apparatus and an image display program that sequentially displays time series images.

BACKGROUND ART

As a technology to grasp contents of time series images that are a series of a plurality of images arranged in a temporal order, such as moving images, in a short time, a technology is known that sets a display time for each frame on the basis of the display importance of each frame that is calculated using data on the amount of brightness variation between frames so that the images are displayed within a length of high-speed display time (see Patent document 1). The high-speed display time length is a period for viewing all the frames at a high speed, and is specified by, for example, a user.

More particularly, firstly, the standard time Std_T for displaying all the frames is calculated by multiplying the number of all the frames N by a standard display time per frame t. The standard display time per frame t is, for example, 1/30 (sec) if NTSC signals are used. An average replay speed Ave_V (double speed) is then calculated by dividing the calculated standard time Std_T by a specified high-speed display time length Set_T. After that, the display importance $A_i$ of each frame (i=1, 2, ..., N) is calculated according to the following equation (1) using inter-frame pixel-based variation amount data DPi (i=1, 2, ..., N) and inter-frame frame-based variation amount data $DF_i$ (i=1, 2, ..., N):

$$A_i = DP_i \times \alpha + DF_i \times \beta \qquad\qquad (1)$$

The coefficients $\alpha$ and that are used for weighting the variation amount data $DP_i$ and $DF_i$ are specified by, for example, the user. Motion of an object in the images and scene changes correspond to the parts to be observed carefully, i.e., parts having high display importance. According to Equation (1), the larger the variation amount data, the higher the calculated display importance becomes.

Display importance Ave_A corresponding to the average replay speed is then calculated by dividing the sum of the display importance of all the frames $\Sigma A_i$ by the number of frames corresponding to the high-speed display time length. The number of frames corresponding to the high-speed display time length is calculated by dividing the number of all the frames N by the average replay speed Ave_V. After that, the display time $T_i$ of each frame (i=1, 2, ..., N) is calculated by dividing the display importance $A_i$ of the corresponding frame by the display importance Ave_A, which corresponds to the average replay speed. The calculation process is represented by the following equation (2):

$$Ti = \frac{Ai}{Ave\_A} = \frac{Ai}{\dfrac{\sum\limits_{i=1}^{N} Ai}{N}} = \frac{Ai}{\dfrac{\sum\limits_{i=1}^{N} Ai}{\dfrac{Std\_T}{Set\_T}}} = \frac{Ai}{\dfrac{\sum\limits_{i=1}^{N} Ai}{\dfrac{N \times t}{Set\_T}}} = \frac{Ai}{\dfrac{\sum\limits_{i=1}^{N} Ai}{\dfrac{Set\_T}{t}}} = \frac{Ai}{\sum\limits_{i=1}^{N} Ai} \times Set\_T \times \frac{1}{t} \quad (i = 1 \ to \ N)$$

$$(2)$$

In the technology of Patent document 1, the display time of each frame is set by dividing the high-speed display time length into segments in accordance with the ratio of the individual frame-based display importance to the sum of the display importance of all the frames, converting each segment into a numerical value that is expressed on the basis of the unit of the standard display time, and allocating the numerical value to the corresponding frame.

Capsule endoscopes that are swallowed through the mouth of a patient, i.e., a subject and then introduced inside the subject, have been proposed in the field of endoscopes. While moving inside the body cavity along, for example, the gullet, the stomach, and the small intestine by peristaltic action, capsule endoscopes sequentially take images of the inner organs and wirelessly send the taken images to a receiving device that is located outside the body. The images of the inner body cavity that are taken by the capsule endoscopes and then received by the receiving device that is located outside the body are displayed sequentially, for example, in the temporal order at a diagnostic workstation or the like to be checked by an observer (user), such as a doctor.

Patent document 1: Japanese Patent No. 2980387

DISCLOSURE OF INVENTION

PROBLEM TO BE SOLVED BY THE INVENTION

If the technology of Patent document 1 is used to display the time series images requiring a long display time, such as the images of the inner body cavity that are taken by capsule endoscopes, it is possible to display images having high display importance long enough to be checked carefully.

The display time appropriate to check images having high display importance varies depending on the user. Suppose, for example, there is a case of checking the images that are taken by the capsule endoscopes. A skilled user can catch important information even when images having high display importance are displayed for a relatively short display time, while a beginner may not catch the important information. The display time that is required for the beginner to fully check each image may be too long for the skilled person and the too-long display time will reduce efficiency. Such a situation will occur not only in cases of observing time series images that are taken by the capsule endoscopes but also in cases of observing some other time series images, such as images taken by surveillance cameras. However, the display time that is calculated using the technology of Patent document 1 is the value that is statically calculated using the ratio of the display importance of the target frame to the sum of the display importance of all the frames, i.e., the display time cannot be specified by the user. Therefore, some users feel that the display time is too long or too short, which leads to overlooking of the display contents or a reduction in the observation efficiency.

The present invention has been achieved to solve the above problems in the conventional technology and it is an object of the present invention to provide an image display apparatus and an image display program that improves, when displaying time series images, observation efficiency, while preventing an overlooking of display contents by the user.

MEANS FOR SOLVING PROBLEM

To solve the problems as described above and to achieve an object, an image display apparatus according to the invention displays time-series images, and the image display apparatus includes a display-condition receiving unit that receives at least an input that specifies a minimum display rate as a display condition for displaying each image that makes up time-series images; an importance calculating unit that calculates an importance coefficient that represents the degree of importance of each of the images; a display-time calculating unit that calculates a display time for each of the images using the display condition that is received by the display-condition receiving unit and the importance coefficient that is calculated by the importance calculating unit; and a display control unit that performs control so that the images are displayed on a display unit sequentially in a temporal order for the respective display times that are calculated by the display-time calculating unit.

Further, an image display program according to the invention causes a computer, which includes a display unit on which time-series images are to be displayed, to execute: a display-condition receiving step of receiving at least an input that specifies a minimum display rate as a display condition for displaying each image that makes up time-series images; an importance calculating step of calculating an importance coefficient that represents a degree of importance of each of the images; a display-time calculating step of calculating a display time for each of the images using the display condition that is received at the display-condition receiving step and the importance coefficient that is calculated at the importance calculating step; and a display control step of performing control so that the images are displayed on the display unit sequentially in a temporal order for the respective display times that are calculated at the display-time calculating step.

EFFECT OF THE INVENTION

In an image display apparatus according to the present invention, because a user can input a condition that defines a minimum display rate that indicates a display time for checking an image having high importance, the image display apparatus can set the display time to a condition of a minimum display rate that varies depending on the user who is observing the time series images. Therefore, it is possible to prevent an overlooking of display contents by the user while increasing the observation efficiency, which makes the user efficiently understand the contents of the time series images.

BRIEF DESCRIPTION OF DRAWINGS

FIG. 1 is a schematic diagram of the general configuration of an image display system including an image display apparatus.

FIG. 2 is an example of an in-vivo image.

FIG. 3 is a block diagram that explains the functional configuration of the image display apparatus.

FIG. 4 is a general flowchart of processes performed by the image display apparatus.

FIG. 5 is an example of a notification screen of an entry request for display conditions.

FIG. 6 is a detailed flowchart of an importance-coefficient calculating process.

FIG. 7 is a detailed flowchart of a display-time calculating process.

FIG. 8 is a graph of a relation between importance coefficient K and display time T.

FIG. 9 is a schematic diagram of an optical flow.

EXPLANATIONS OF LETTERS OR NUMERALS

| 10 | Capsule endoscope |
|---|---|
| 30 | Receiving device |
| A1 to An | Receiving antennas |
| 50 | Portable recording medium |
| 70 | Image display apparatus |
| 710 | Image obtaining unit |
| 720 | Input unit |
| 730 | Display unit |
| 740 | Storage unit |
| 741 | Display-condition setting data |
| 743 | Importance-coefficient data |
| 745 | Display-time data |
| 747 | Image display program |
| 750 | Calculating unit |
| 751 | Importance-coefficient calculating unit |
| 751a | Inter-image variation amount calculating unit |
| 751b | Target-object extracting unit |
| 753 | Display-time calculating unit |
| 760 | Control unit |
| 761 | Image-display-condition setting unit |
| 763 | Image-display control unit |
| 1 | Subject |

BEST MODE(S) FOR CARRYING OUT THE INVENTION

Exemplary embodiments of the present invention are described in detail below with reference to the accompanying drawings.

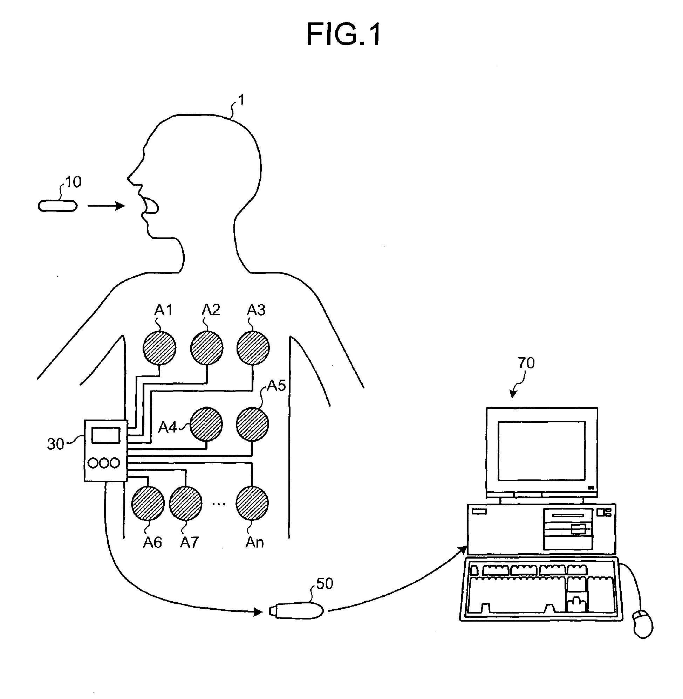

FIG. 1 is a schematic diagram of a general configuration of an image display system including an image display apparatus according to an embodiment of the present invention. The image display apparatus according to the present embodiment is an image display apparatus that displays images of an inner body cavity that are taken by a capsule endoscope. As shown in FIG. 1, the image display system includes, for example, a capsule endoscope 10 that takes images of an inside of a subject 1 (in-vivo images); a receiving device 30 that receives the in-vivo images wirelessly from the capsule endoscope 10; and an image display apparatus 70 that displays the in-vivo images that are taken by the capsule endoscope 10 using the in-vivo images received by the receiving device 30. A recording medium that can be carried (portable recording medium) 50, for example, is used to pass image data between the receiving device 30 and the image display apparatus 70.

The capsule endoscope 10 has an imaging function and a wireless communication function, etc. After swallowed through the mouth of the subject 1 and introduced inside the subject 1, the capsule endoscope 10 sequentially takes the in-vivo images, while moving inside the body cavity. The capsule endoscope 10 then wirelessly sends the taken in-vivo images outside the body. The imaging rate of the taken in-vivo images is 2 to 4 (frames/sec) and several tens of thousands of in-vivo images are taken while the capsule endoscope 10 is inside the body for about 8 hours (8x60x60 sec). Because the moving speed of the capsule endoscope 10 inside the body cavity is not constant, change in the taken image over the time series frames is various. The image may show no change over several hundreds of frames, while the image may show change one frame by one frame.

The receiving device 30 includes a plurality of receiving antennas A1 to An and wirelessly receives the in-vivo images from the capsule endoscope 10 via the receiving antennas A1 to An. The receiving device 30 is configured so that the portable recording medium 50 can be attached/detached thereto/therefrom and sequentially stores the image data of the received in-vivo images in the portable recording medium 50. In this manner, the receiving device 30 stores the in-vivo images, which are the images of inside of the subject 1 taken by the capsule endoscope 10, in the portable recording medium 50 in the time-series order.

The receiving antennas A1 to An are, for example, loop antennas and arranged, as shown in FIG. 1, at predetermined positions, spaced from each other, on the surface of the subject 1. More particularly, for example, the receiving antennas A1 to An are arranged at the positions, spaced from each other, corresponding to a path along which the capsule endoscope 10 moves inside the subject 1. The receiving antennas A1 to An can be arranged spaced from each other on a jacket which the subject 1 wears. In such a case, when the subject 1 wears the jacket, the receiving antennas A1 to An are arranged at the predetermined positions on the surface of the subject 1 corresponding to the path along which the capsule endoscope 10 moves inside the subject 1. The number of the receiving antennas is not limited, as long as at least one receiving antenna is arranged on the subject 1.

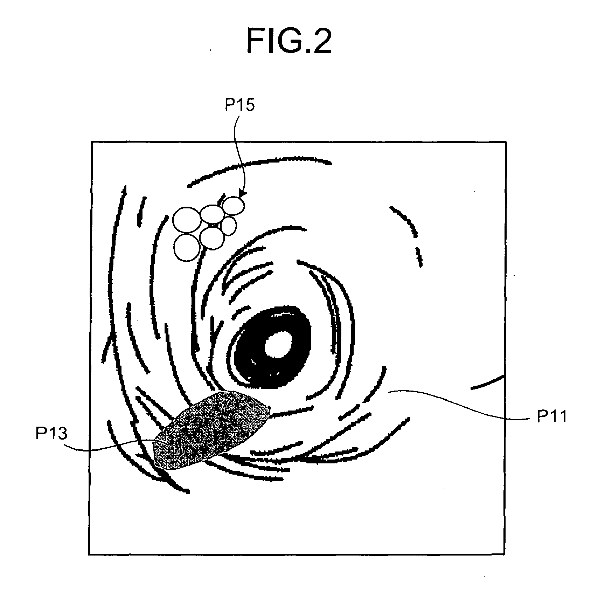

The image display apparatus 70 is a general-purpose computer, such as a workstation and a personal computer, and is configured so that the portable recording medium 50 can be attached/detached thereto/therefrom. The image display apparatus 70 obtains the time series in-vivo images stored in the portable recording medium 50 and displays the obtained time series in-vivo images sequentially by switching one from another on a display, such as an LCD and an ELD. FIG. 2 is an example of the in-vivo image that is taken by the capsule endoscope 10 and then displayed by the image display apparatus 70. The in-vivo image includes, for example, a mucosa membrane P11, a content P13 that is floating inside the body cavity, and a bubble P15. Some in-vivo images include an important part such as lesions. The in-vivo images taken by the capsule endoscope 10 are color images including pixels each having a pixel level (pixel value) corresponding to each of the color components R (red), G (green), and B (blue).

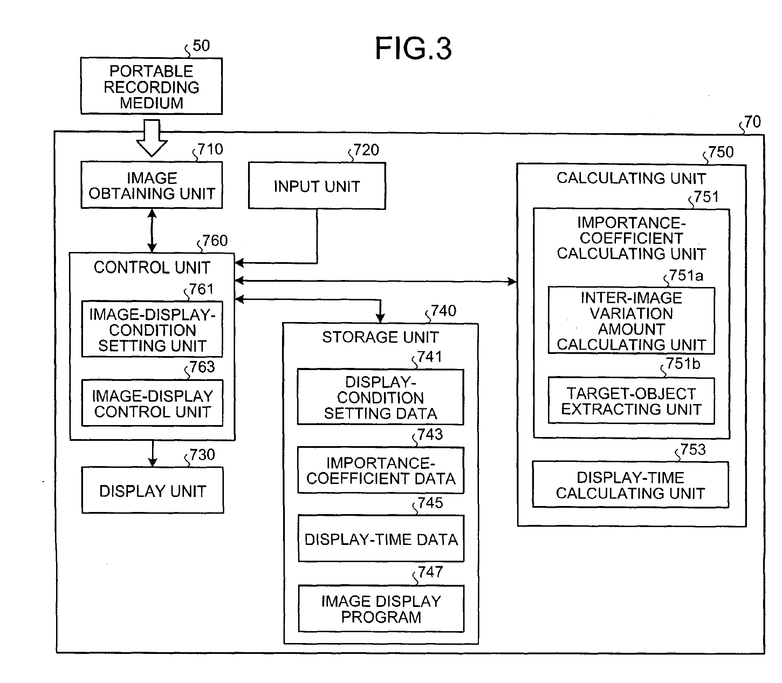

FIG. 3 is a block diagram that explains the functional configuration of the image display apparatus 70. The image display apparatus 70 according to the present embodiment includes an image obtaining unit 710, an input unit 720, a display unit 730, a storage unit 740, a calculating unit 750, and a control unit 760 that controls the units of the device.

The image obtaining unit 710 is a functional unit corresponding to an image obtaining unit that obtains the time series in-vivo images that are taken by the capsule endoscope 10. The image obtaining unit 710 is attached with the portable recording medium 50, for example, and reads image data of some of the time series in-vivo images that are stored in the portable recording medium 50, thereby obtaining target in-vivo images to be displayed. The image obtaining unit 710 is implemented by, for example, a reading/writing device in accordance with a type of the portable recording medium 50. Alternatively, the image display system may be configured to include a hard disk in place of the portable recording medium 50 and the image obtaining unit 710, and the time series images may be pre-stored (the time series in-vivo images in the present embodiment) in the hard disk. Still alternatively, a server can be used instead of the portable recording medium 50. In such a case, the time series images are pre-stored in the server. The image display apparatus 70 obtains time series images from the server by connecting to the server via the image obtaining unit. In this case, the image obtaining unit is implemented by a communication device or the like that is connected to the server.

The input unit 720 is implemented by, for example, a keyboard, a mouse, a touch panel, and various switches, and outputs operation signals to the control unit 760 in accordance with operation inputs. The display unit 730 is implemented by a display device, such as an LCD and an ELD and displays various screens including a display screen of the time series in-vivo images by the operation of the control unit 760.

The storage unit 740 is implemented by an information recording medium, a corresponding reading device, etc, for example, by various IC memories, such as a ROM or a RAM that is an updatable flash memory, a hard disk that is built-in or connected using a data communication terminal, and a CD-ROM. The storage unit 740 stores therein programs that implement various functions of the image display apparatus 70 and data that is used to implement these programs. The storage unit 740 stores therein display-condition setting data 741 that includes display conditions that are set by a later-described image-display-condition setting unit 761 to display the in-vivo images; importance-coefficient data 743 that includes an importance coefficient of each of the in-vivo images that is calculated by an importance-coefficient calculating unit 751; display-time data 745 that includes the display time for each of the in-vivo images that is calculated by a display-time calculating unit 753; and an image display program 747 that causes the display unit 730 to sequentially display the time series in-vivo images.

The calculating unit 750 is implemented by a hardware, such as a CPU, and performs various calculation processing to cause the display unit 730 to display the target time series in-vivo images that are obtained by the image obtaining unit 710 for the display time in accordance with the importance. The calculating unit 750 includes the importance-coefficient calculating unit 751 that calculates the importance coefficient that represents a degree of importance of each of the in-vivo image to be displayed; and the display-time calculating unit 753 that calculates the display time for each of the in-vivo images. The importance-coefficient calculating unit 751 includes an inter-image variation amount calculating unit 751a that calculates an inter-image variation amount between the in-vivo images that are continuous in terms of the temporal order; and a target-object extracting unit 751b that extracts a target object from each of the in-vivo images.

The control unit 760 is implemented by a hardware, such as a CPU. The control unit 760 sends instructions or data to a target unit based on the image data that is received from the image obtaining unit 710, the operation signal that is received from the input unit 720, programs and data that are stored in the storage unit 740, etc., thereby controlling the operation of the image display apparatus 70. The control unit 760 includes the image-display-condition setting unit 761 that sets the display conditions concerning the time series in-vivo images that are obtained by the image obtaining unit 710 according to the user operation received via the input unit 720; and an image-display control unit 763 that performs control so that the in-vivo images are displayed on the display unit 730 sequentially in the temporal order for the respective display times.

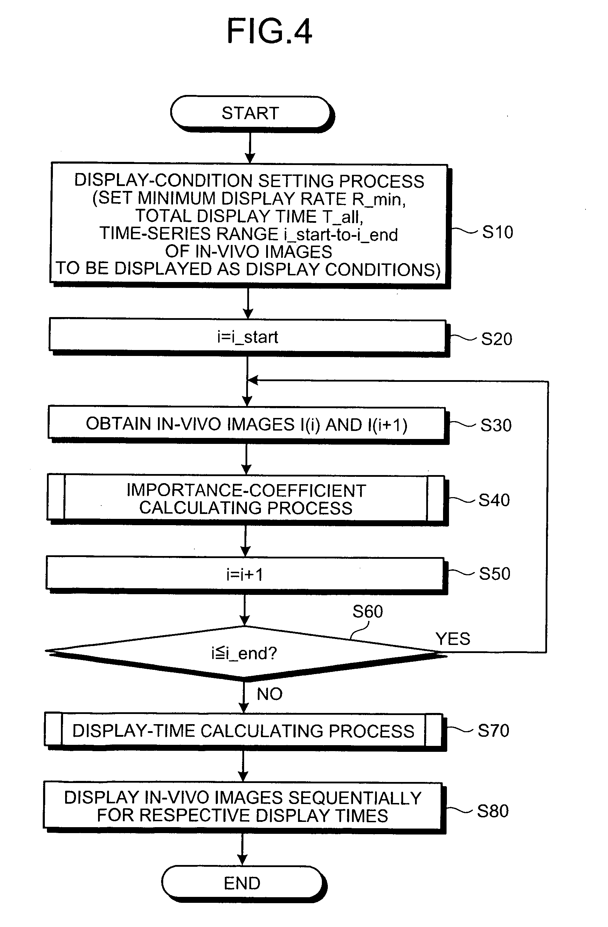

FIG. 4 is a general flowchart of processes performed by the image display apparatus 70. The processes that are described below are implemented when the units of the image display apparatus 70 operates, following the image display program 747.

As shown in FIG. 4, the image-display-condition setting unit 761 first performs a display-condition setting process, thereby setting a minimum display rate R_min, a total display time T_all that it takes to display all the in-vivo images to be displayed, and a time-series range i_start-to-i_end of the in-vivo images to be displayed as the display conditions (Step S10), in which i_start indicates the image number of the top image of the in-vivo images to be displayed within the time-series range and i_end indicates the image number of the last image of the in-vivo images to be displayed within the time-series range. The image numbers of the in-vivo images represent the temporal order of the in-vivo images. The image numbers can be, for example, assigned in the receiving order when the receiving device 30 stores the received in-vivo images in the portable recording medium 50 or in the storing order when the image display apparatus 70 obtains the in-vivo images from the portable recording medium 50.

The calculating unit 750 then initializes the image number i, which indicates the temporal order of the in-vivo images to be processed, to "i_start" (Step S20). After that, the calculating unit 750 obtains an in-vivo image I(i) that is an in-vivo image to be processed and an in-vivo image I(i+1) that is an in-vivo image that is continuous to the in-vivo image to be processed in terms of the temporal order (Step S30).

After that, the importance-coefficient calculating unit 751 performs an importance-coefficient calculating process (Step S40). After the importance-coefficient calculating process at Step S40, the calculating unit 750 increments the image number i, which indicates the temporal order, i.e., sets i=i+1 (Step S50) and determines whether the next in-vivo image I(i) to be processed is present using i≤i_end. If i≤i_end (Step S60: Yes), the processes from Step S30 to Step S50 are repeated. On the other hand, if i>i_end (Step S60: No), the process control goes to Step S70 and the display-time calculating unit 753 performs a display-time calculating process. After that, the image-display control unit 763 performs control so that the target in-vivo images that are present within the time-series range i_start-to-i_end are displayed on the display unit 730 sequentially in the temporal order for the respective display times (Step S80).

The display-condition setting process at Step S10 of FIG. 4 is described below. The image-display-condition setting unit 761 sets, by the user operations, various display conditions including the minimum display rate R_min, the total display time T_all, and the time-series range i_start-to-i_end within which the in-vivo images to be displayed are present. More particularly, the image-display-condition setting unit 761 causes the display unit 730 to display a notification of an entry request for the display conditions and then receives inputs that specify the display conditions via the input unit 720. The image-display-condition setting unit 761 works as a display-condition receiving unit through displaying the notification of the entry request for the display conditions on the display unit 730 and receiving the inputs of the display conditions via the input unit 720.

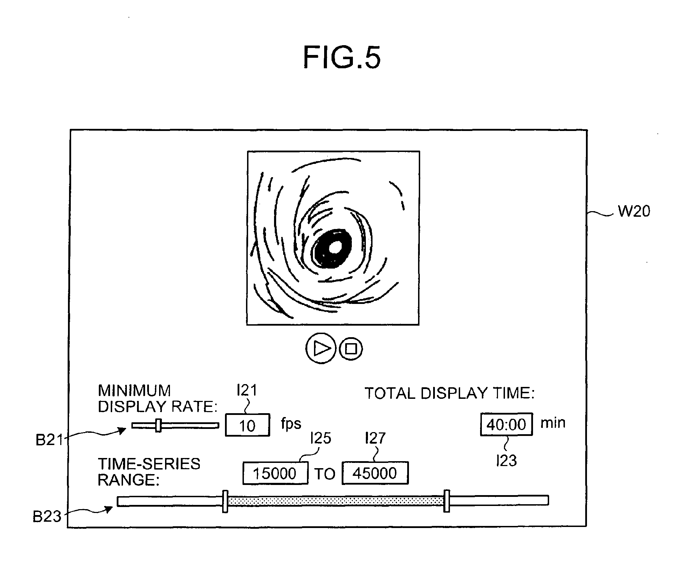

FIG. 5 is an example of a notification screen W20 of an entry request for the display conditions. The notification screen W20 includes an entry box I21 and a slide bar B21 that receive an input operation of the minimum display rate R_min, an entry box I23 that receives an input operation of the total display time T_all, and an entry boxes I25 and I27 and a slide bar B23 that receive an input operation of the time-series range i_start-to-i_end of the in-vivo images to be displayed. The user enters, via the input unit 720, the desired value of the minimum display rate R_min to the entry box I21 or indirectly to the entry box I21 using the operation of the slide bar B21. The user enters the desired value of the total display time T_all to the entry box I23. The user enters the image number i_start of the top image within the desired time-series range to the entry box I25 and the image number i_end of the last image to the entry box I27 or indirectly to the entry boxes I25 and I27 using the operation of the slide bar B23. The image-display-condition setting unit 761 sets the display conditions, following the contents of the response to the notification of the entry request. Data of the specified display conditions are stored in the storage unit 740 as the display-condition setting data 741. It is allowable to receive an input that indirectly specifies the value of the minimum display rate R_min and the value of the total display time T_all instead of the input that directly specifies the value of the minimum display rate R_min and the value of the total display time T_all. For example, it can be configured to receive options including "fast", "normal", "slow" as the input to specify the minimum display rate R_min indirectly. By setting the value of the minimum display rate R_min corresponding to each option to a predetermined value, the user enters the value of the minimum display rate R_min indirectly.

In this manner, according to the present embodiment, the display conditions including the minimum display rate R_min are set by the user operation. The display rate in the description corresponds to the display time per in-vivo image. The display time increases as the display rate decreases, while the display time decreases as the display rate increases. That is, the minimum display rate R_min that is set by the user operation is the maximum display time for one in-vivo image (maximum display time). The user specifies the maximum display time for the in-vivo image by setting the minimum display rate R_min. With this configuration, if the user is a skilled user, for example, the user sets the maximum display time short, or if the user is a beginner, the user sets the maximum display time long, i.e., the user can adjust the maximum display time, appropriately.

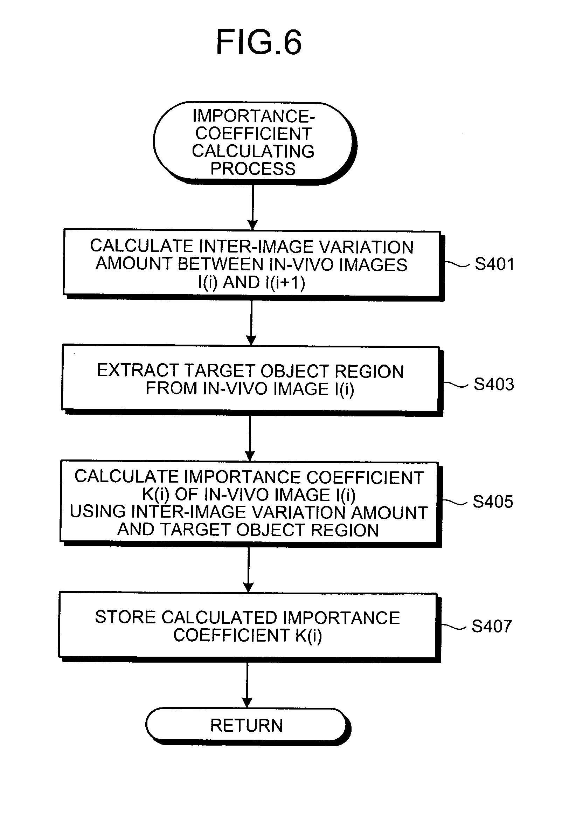

The importance-coefficient calculating process at Step S40 of FIG. 4 is described below. FIG. 6 is a detailed flowchart of the importance-coefficient calculating process.

The inter-image variation amount calculating unit 751a first calculates the inter-image variation amount between the in-vivo image I(i) and the in-vivo image I(i+1) (Step S401). For example, the inter-image variation amount calculating unit 751a calculates the inter-image variation amount using a degree of similarity between the in-vivo image I(i) and the in-vivo image I(i+1). More particularly, a sum of squared differences SSD between the pixel value of each pixel in the in-vivo image I(i) and the pixel value of the corresponding pixel in the in-vivo image I(i+1) is calculated using the following equation (3):

$$ SSD = \sum_{y=0}^{Y-1} \sum_{x=0}^{X-1} \left( P_{I(i+1)}(x, y) - P_{I(i)}(x, y) \right)^2 \qquad (3) $$

where $P_{I(i)}(x, y)$ and $P_{I(i+1)}(x, y)$ are pixel values of the in-vivo image I(i) and the in-vivo image I(i+1), respectively; X and Y are a length of the image in the x direction and a length of the image in the y direction, respectively. Because the in-vivo images have a pixel value corresponding to each color component of RGB, the value of the SSD of each color component is calculated. A sum or an average of the color-component-based SSDs is then calculated, and the obtained value is assumed to be the inter-image variation amount. As for the in-vivo image that is at the end of the time-series range, the inter-image variation amount is calculated using the continuous in-vivo image therebefore in the temporal order.

After that, the target-object extracting unit 751b extracts a target object region from the in-vivo image I(i) (Step S403). The target object in the in-vivo image is, for example, affected sites. There have been various known methods of extracting regions in which these affected sites are present (target object regions). For example, the target object region in which an affected site is present is extracted using the color data of each pixel. This extracting method is disclosed in, for example, Japanese Patent Application Laid-open No. 2005-192880. More particularly, firstly, each pixel in the in-vivo image I(i) or the average of the pixel values are mapped on a feature space based on the color data. Healthy mucosa-membrane clusters and affected-site clusters are then identified through clustering of each pixel or the average of the pixels values in the feature space, and a pixel region that belongs to the affected-site cluster is extracted as the target object region in which the affected site appears. The mucosa membrane in the body cavity can be assumed to be the target object. Although contents and bubbles inside the body cavity can be present in the in-vivo image, an object to be checked in the test is mainly the mucosa membrane. Therefore, a region of the mucosa membrane in the in-vivo image can be extracted as the target object region. It is allowable to extract the target object region using the color data of each pixel or by identifying regions of the mucosa membrane, the contents, and the bubbles in the in-vivo image and then extracting the regions determined to be the mucosa membrane. The methods of extracting the target object are not limited thereto and any extracting methods can be used appropriately in accordance with a type of the target object.

After that, the importance-coefficient calculating unit 751 calculates an importance coefficient K(i) of the in-vivo image I(i) using the inter-image variation amount that is calculated at Step S401 and the target object region that is extracted at Step S403 (Step S405). The importance-coefficient calculating unit 751 calculates the importance coefficient K(i) using, for example, the following equation (4) that is a linear combination equation:

$$ K(i) = coeff1 \times Diff + cieff2 \times Detect \qquad (4) $$

where Diff is the inter-image variation amount; Detect is target object data that is decided based on a result of the extracted target object region. In a case where, for example, presence of the target object region is used as the target object data, if the target object region is extracted from the in-vivo image I(i), the value of Detect is "0 (non presence)", while if no target object region is extracted, the value of Detect is "1 (presence)". It is allowable to use a presence ratio of the target object to the in-vivo image I(i) as the target object data. The parameters coeff1 and coeff2 are weighting coefficients that are set to predetermined values. Because the in-vivo image that is remarkably different from the continuous images or the images arranged near in the temporal order, and the image including the target object are highly important, coeff1 and coeff2 are positive coefficients, in general.

The importance-coefficient calculating unit 751 then sets the calculated importance coefficient K(i) to the importance-coefficient data 743 associated with the image number i of the in-vivo image I(i) and stores it in the storage unit 740 (Step S407). After that, the process control returns to Step S40 of FIG. 4.

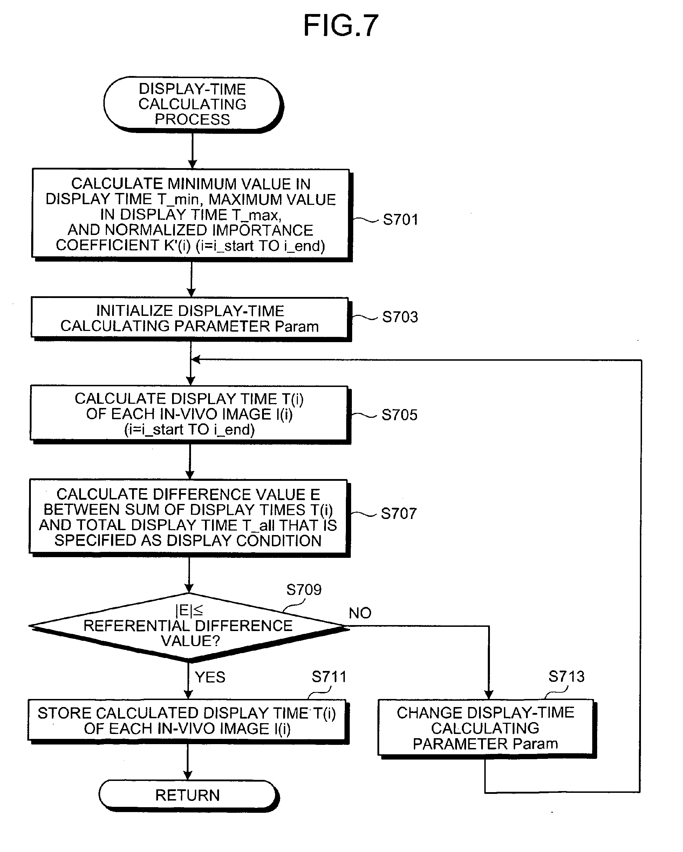

The display-time calculating process at Step S70 of FIG. 4 is described below. FIG. 7 is a detailed flowchart of the display-time calculating process.

The display-time calculating unit 753 first calculates the minimum display time $T\_min$ of the in-vivo image, the maximum display time $T\_max$, and the normalized importance coefficient K'(i) (Step S701).

The minimum display time $T\_min$ of the in-vivo image is calculated using the value of the maximum display rate $R\_max$, which is the upper limit of the display speed of the display unit 730. The value of the maximum display rate $R\_max$ is determined by performances of the hardware, etc; however, any value that is the upper limit or lower can be set by the user operation. More particularly, the minimum display time $T\_min$ of the in-vivo image is calculated using the following equation (5):

$$T\_\min = \frac{1}{R\_\max} \qquad (5)$$

The maximum display time $T\_max$ of the in-vivo image is calculated using the minimum display rate $R\_min$ that is set in the display-condition setting process by the user operation. More particularly, the maximum display time $T\_max$ of the in-vivo image is calculated using the following equation (6):

$$T\_\max = \frac{1}{R\_\min} \qquad (6)$$

The normalized importance coefficient K'(i) that is obtained by normalizing the importance coefficient K(i) of the in-vivo image I(i) (i=i_start to i_end) within a range from 0 to 1 is calculated using the following equation (7) in which $K\_max$ is the maximum value of the importance coefficient and is predetermined.

$$K'(i) = \begin{cases} \dfrac{K(i) - \min_i(K(i))}{\max_i(K(i)) - \min_i(K(i))} & : (K(i) < K\_\max) \\ \\ 1 & : (K(i) \geq K\_\max) \end{cases} \qquad (i = i\_start \ to \ i\_end) \qquad (7)$$

If K(i) is equal to or larger than $K\_max$, which is a threshold, the value of K'(i) is set to "1". If the value of K'(i) is set to "1", the display time for the in-vivo image I(i) is the maximum display time $T\_max$ according to the following equation (8). As a result, the display time for the in-vivo image having a high degree of importance is set to the maximum display time $T\_max$.

After that, the display-time calculating unit 753 initializes a display-time calculating parameter param (Step S703). For example, the display-time calculating unit 753 initializes the display-time calculating parameter param to the default value of "1". The display-time calculating unit 753 then calculates the display time T(i) of each of the in-vivo images I(i) (i=i_start to i_end) using the following equation (8) (Step S705):

$$T(i) = \left(T\_\max - T\_\min\right) \times \left(K'(i)\right)^{param} + T\_\min \quad \left(i = i\_start\ to\ i\_end\right) \quad (8)$$

The function expression (8) that is used to calculate the display time T(i) is an example, and not limiting.

After that, the display-time calculating unit 753 calculates a difference value E between the sum of the display times T(i) of the in-vivo images I(i) that are calculated at Step S705 and the total display time T_all that is set in the display-condition setting process by the user operation using the following equation (9) (Step S707):

$$E = T\_all - \sum_{i=i\_start}^{i\_end} T(i) \quad\quad (9)$$

The display-time calculating unit 753 then compares the difference value E that is calculated at Step S707 with a referential difference value that is set as the threshold, thereby determining whether the difference between the sum of the display times T(i) of the in-vivo images I(i) and the total display time T_all is small enough to accept. If the absolute value of the calculated difference value E is equal to or smaller than the referential difference value, the display-time calculating unit 753 determines that the difference between the sum of the display times T(i) of the in-vivo images I(i) and the total display time T_all is acceptable (Step S709: Yes), creates the display-time data 745 by associating the calculated display time T(i) of each of the in-vivo images I(i) with the image number i of the corresponding in-vivo image I(i), and stores the display-time data 745 in the storage unit 740 (Step S711). After that, the process control returns to Step S70 of FIG. 4.

On the other hand, If the absolute value of the calculated difference value E is larger than the referential difference value, the display-time calculating unit 753 determines that the difference is not acceptable (Step S709: No), changes the value of the display-time calculating parameter param (Step S713), and performs the processes from Steps S705 to S709, again.

When the absolute value of the calculated difference value E is larger than the referential difference value, the display-time calculating parameter param is changed to a value that is calculated using the following equation (10) in which dp is an updating coefficient, having a positive value:

$$param = param \times (1 - dp \times E) \quad\quad (10)$$

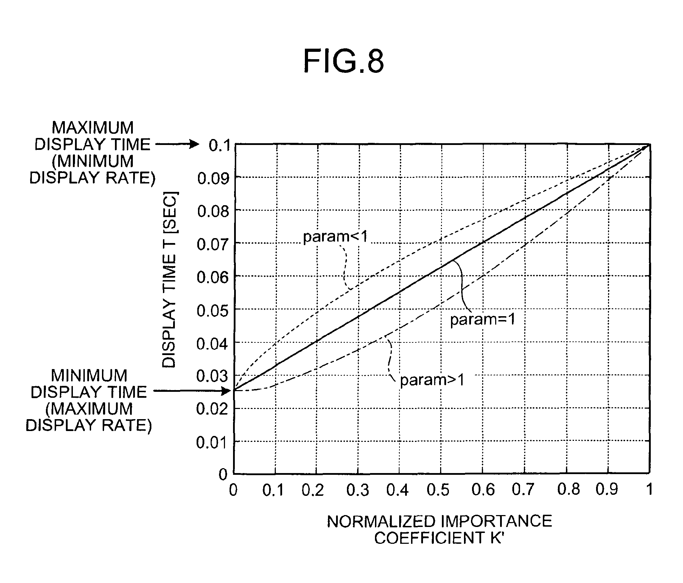

A relation between the importance coefficient K' that is obtained by normalizing the importance coefficient K that is used in Equation (8) and the display time T is described below. FIG. 8 is a graph of the relation between the importance coefficient K' and the display time T. As shown in FIG. 8, if the display-time calculating parameter param=1, the relation between the importance coefficient K' and the display time T is expressed by the increasing function that is indicated by the continuous line. If the sum of the display times T(i) of the in-vivo images I(i) that is calculated using this relation is smaller than the total display time T_all (E>0), it is necessary to increase the display times T(i) of the in-vivo images I(i) in whole. If param<1, the relation between the importance coefficient K' and the display time T is expressed by the increasing function, indicated by the broken line, that protrudes toward the upper left side of FIG. 8. If the display time T(i) of each in-vivo image I(i) is calculated using this relation, the display times T(i) of the in-vivo images I(i) are increased as compared with param=1. On the other hand, if the sum of the display times T(i) of the in-vivo images I(i) that is calculated using this relation is larger than the total display time T_all (E<0), it is necessary to decrease the display times T(i) of the in-vivo images I(i) in whole. If param>1, the relation between the importance coefficient K' and the display time T is expressed by the increasing function, indicated by the dashed-dotted line, that protrudes toward the lower right side of FIG. 8. If the display time T(i) of each in-vivo image I(i) is calculated using this relation, the display times T(i) of the in-vivo images I(i) are decreased in whole as compared with param=1. The display time T(i) of each in-vivo image I(i) is calculated again in this manner, and the value of the display-time calculating parameter param is adjusted (changed) in accordance with the difference value E between the sum of the display times T(i) of the in-vivo images I(i) and the total display time T_all, whereby the display time T(i) of each in-vivo image I(i) is calculated so that the sum comes close to the total display time T_all in a numerical-analytic manner.

In some cases, the sum of the display times T(i) of the in-vivo images I(i) cannot come close to the total display time T_all even when param is changed in the above manner, and the display time T(i) of each in-vivo image I(i) cannot be determined. If, for example, the sum of the display times T(i) is calculated in the conditions that the display time T(i) of the in-vivo image I(i) having the importance coefficient=0 is set to the minimum display time T_min, while the display time T(i) of the in-vivo image I(i) having the importance coefficient≠0 is set to the maximum display time T_max, and the total display time T_all that is larger than the sum of the display times T(i) is set, the sum of the display times T(i) may not come close to the total display time T_all even if the value of param is small enough. Moreover, if the sum of the display times T(i) is calculated in the conditions that the display time T(i) of the in-vivo image I(i) having the importance coefficient=0 is set to the maximum display time T_max, while the display time T(i) of the in-vivo image I(i) having the importance coefficient≠0 is set to the minimum display time T_min, and the total display time T_all that is smaller than the sum of the display times T(i) is set, the sum of the display times T(i) may not come close to the total display time T_all even if the value of param is large enough. In the latter case, it is allowable to reduce the number of images to be displayed by setting an interval of an in-vivo image I(i) having a small importance coefficient K(i), i.e., low importance to "0" in a skipping process and then re-calculate the display time T(i) of each of the in-vivo images I(i) to be displayed. If the display time T(i) of each of the in-vivo images I(i) cannot be determined even using this result, it is allowable to request the user for correction by causing the image-display-condition setting unit 761 to display a warning message on the display unit 730, saying that the total display time T_all is too small. It is also allowable, in the former case, to request the user for correction by causing the image-display-condition setting unit 761 to display a warning message on the display unit 730, saying that the total display time T_all is too large.

After that, the image-display control unit 763 refers to the display-time data 745 that is created as the result of the display-time calculating process and causes the display unit 730 to display the in-vivo images within the time-series range for the respective display times sequentially in the temporal order at Step S80 of FIG. 4. In other words, the image-display control unit 763 first displays the in-vivo image I(i_start) having the image number i_start for the display time T(i_start) and then displays the next in-vivo image I(i_start+1) having the image number i_start+1 for the display time T(i_start+1), and so on. In this manner, the image-display control unit 763 causes the display unit 730 to sequentially display the in-vivo images up to the in-vivo image I(i_end) within the total display time corresponding to the total display time T_all. In reading of the in-vivo images to be displayed from the portable recording medium 50, it is allowable to read either all the in-vivo images to be displayed at one time or sets of a predetermined number of images one after another.

As it has been mentioned, according to the present embodiment, the display conditions can be specified to display a plurality of time-series in-vivo images that are taken by the capsule endoscope 10. The display conditions are the minimum display rate that corresponds to the maximum display time per in-vivo image, the total display time for displaying all the target in-vivo images, and the time-series range of the in-vivo images to be displayed. Because the user specifies the maximum display time to a desired value appropriate for the user who is observing the images, an overlooking of the display contents by the user is prevented and the observation efficiency is improved, and thus the user efficiently understands the contents of the plurality of images that are continuous in the temporal order.

Moreover, the image display apparatus calculates the inter-image variation amount between the in-vivo images that are continuous in the temporal order, extracts the target object region from each of the in-vivo images, and calculates the importance coefficient of each in-vivo image using the calculated inter-image variation amount and the result of the extracted target object region. The importance of the image is calculated using a change in the brightness in Patent document 1 that is described in the background art; however, even if a change in the brightness is large, the importance of the image in which the target object is not present is low. Therefore, it is not always appropriate to display the images according to the importance determined based on the changes in brightness. In the present embodiment, because the importance coefficient is calculated using presence of the target object in the image, the importance of each in-vivo image is determined appropriately.

Furthermore, the image display apparatus calculates the display time for each in-vivo image on the basis of the specified display conditions and the calculated importance coefficient, and displays the images on the display unit sequentially in the temporal order for the respective display times. With this configuration, it is possible to provide an image display apparatus that sets the maximum display time, which is the display time for a highly important image, and displays the in-vivo images for the desired total display time in accordance with their importance.

Although, in the present embodiment, the inter-image variation amount calculating unit 751a calculates the sum of squared differences SSD between the pixel value of each pixel in the in-vivo image I(i) and the pixel value of the corresponding pixel in the in-vivo image I(i+1) using Equation (3) and then calculates the inter-image variation amount using the value of the calculated SSD, it is allowable to calculate a sum of absolute differences SAD between the pixel value of each pixel in the in-vivo image I(i) and the pixel value of the corresponding pixel in the in-vivo image I(i+1) using the following equation (11) and then calculate the inter-image variation amount using the value of the calculated SAD:

$$SAD = \sum_{y=0}^{Y-1} \sum_{x=0}^{X-1} \left| P_{I(i+1)}(x, y) - P_{I(i)}(x, y) \right| \qquad (11)$$

Alternatively, it is allowable to calculate a normalized cross-correlation NCC of the in-vivo images I(i) and I(i+1) using the following equation (12) and then calculate the inter-image variation amount using the value of the calculated NCC. The value of the NCC is from -1 to 1, where the value increases as the degree of change between images decreases. Therefore, it is necessary to perform a process, such as inverting the symbol.

$$NCC = \frac{\sum\limits_{y=0}^{Y-1} \sum\limits_{x=0}^{X-1} P_{I(i+1)}(x, y) \times P_{I(i)}(x, y)}{\sqrt{\sum\limits_{y=0}^{Y-1} \sum\limits_{x=0}^{X-1} P_{I(i+1)}(x, y)^2 \times \sum\limits_{y=0}^{Y-1} \sum\limits_{x=0}^{X-1} P_{I(i)}(x, y)^2}} \qquad (12)$$

The manner of calculating the inter-image variation amount is not limited to the manners using the degree of similarity between the images. The inter-image variation amount can be calculated using, for example, an optical flow. The optical flow is a flow that is obtained by mapping the same object that is present in two images that are taken at different time points and then expressing its moving amount as vector data. To calculate the inter-image variation amount using this method, the in-vivo image I(i) as a calculation target is first divided into segments having a predetermined size in a grid pattern. Each segment is then set to be a template sequentially, and a correspondence area that is similar to each template is detected from the in-vivo image I(i+1). As the template matching technique, for example, the technique disclosed in "Template Matching, 202 p, Digital Image Processing, Computer Graphic Arts society" can be used. A search area for the matching can be defined by setting the central coordinates in each template to the center, taking, for example, the moving amount of the target object to be imaged into consideration. Moreover, some other techniques, such as a coarse-to-fine search and a sequential similarity detection algorism, can be used to increase the speed. For example, the technique disclosed in "High-speed Search, 206 p, Digital Image Processing, Computer Graphic Arts Society" can be used.

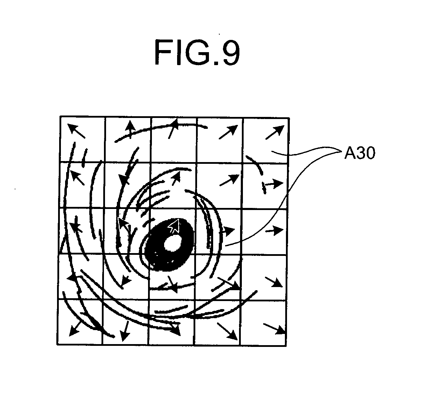

As a result, the coordinates and the degree of similarity of the correspondence area that is most similar to each template that is corresponding to the segment is acquired from the in-vivo image I(i+1). The optical flow of each segment of the in-vivo image I(i) is created using the coordinates and the degree of similarity of the acquired correspondence area. In other words, the optical flow is calculated by calculating a vector from the central coordinates of each segment of the in-vivo image I(i) to the central coordinates of the correspondence area. FIG. 9 is a schematic diagram of the optical flow. As shown in FIG. 9, the optical-flow vector of each segment A30 from the central coordinates is calculated. After that, the maximum length or the average of the lengths of the segment-based optical-flow vectors from the central coordinates is calculated, and the calculated value is set to the inter-image variation amount. It is allowable to exclude a template that is determined at the matching to have a low degree of similarity from the targets that are subjected to the calculation of the inter-image variation amount, because there is a high possibility that such a template has no association with the same object. Moreover, it is allowable to set only a part of the segment to be the template instead of setting the entire area of the segment. Still moreover, it is not necessary to set all the segments to the template for detection of the correspondence area. It is allowable to set some selected segments to be the template for detection of the correspondence area. In a case where the capsule endoscope 10 moves inside the body cavity, an object that is present in the edge part of the in-vivo image I(i) usually goes out of the field of view in the in-vivo image I(i+1). Therefore, it is allowable to define an area that is to be set as the template by excluding the edge part from the in-vivo image I(i).

Alternatively, the inter-image variation amount can be calculated using changes of amounts of statistics. The amounts of statistics related to the images are, for example, an average, a variance, and a histogram of the pixel values of respective pixels that make up the image. In this case, firstly, a value of an amount of statistics Stat between the in-vivo image I(i) and the in-vivo image I(i+1) is calculated. A difference D_Euclid in the amount of statistics between the images is then calculated using the following equation (13), and the calculated value is set to the inter-image variation amount. If the amount of statistics to be calculated is the average or the variance of the pixel values, the value of the amount of statistics Stat is calculated on the basis of each of the RGB color components so that three-dimensional data is obtained. If the amount of statistics to be calculated is the histogram, the histogram is calculated on the histogram divided number n_edge basis so that $3 \times$ n_edge dimensional data is obtained.

$$D\_Euclid = \sqrt{\sum_{d=1}^{D} \left( Stat_{I(i+1)}^{d} - Stat_{I(i)}^{d} \right)^2} \qquad (13)$$

where $Stat_{I(i)}^{d}$ is a d-th value of a D-dimensional amount of statistics of the in-vivo image I(i), in which d (d=1 to D) is the dimension number.

It is allowable to calculate a multidimensional feature vector that is a combination of several types of the amounts of statistics including the average, the variance, and the histogram, etc., and calculate the difference in the amount of statistics between the in-vivo image I(i) and the in-vivo image I(i+1) using the multidimensional feature vector. Moreover, in the calculation, it is allowable to normalize the values of the amounts of statistics or perform weighting by multiplying the numerical value of each amount of statistics by a predetermined weighting coefficient.

Although all the RGB color components are used to calculate the inter-image variation amount, the inter-image variation amount can be calculated using only a color component that represents changes in the images clearly. For example, in the in-vivo images taken by the capsule endoscope 10, among the values of the RGB components, the G value tends to represent changes between the images clearly, because the G value is close to the absorption band (wavelength) of hemoglobin in blood, and has a high sensitivity and a high resolution. Therefore, it is possible to calculate the inter-image variation amount using the G value.

It is also allowable to calculate the inter-image variation amount using information that is calculated as two-dimensional data using the pixel values with a well-known conversion technique. For example, the inter-image variation amount can be calculated using the brightness and the color difference that are calculated by an YCbCr conversion, or the hue, the color saturation, and the lightness, etc., that are calculated by an HIS conversion.

In the above embodiment, the inter-image variation amount between the in-vivo image I(i) and the in-vivo image I(i+1) is calculated; however, it is not always necessary to calculate the inter-image variation amount between sequential images. For example, it is allowable to select images close to each other in the temporal order as appropriate, and calculate the inter-image variation amount between the selected images. Moreover, it is allowable to calculate variation amounts between the in-vivo image I(i) and a plurality of in-vivo images near the in-vivo image I(i) in the temporal order and set the average of the calculated values to be the inter-image variation amount of the in-vivo image I(i).

Some methods other than the methods of calculating the inter-image variation amount can be used as long as a numerical value corresponding to a change between the in-vivo image I(i) and the in-vivo image I(i+1) is obtained.

The equation that is used to calculate the importance coefficient K(i) by the importance-coefficient calculating unit 751 is not limited to the linear combination equation indicated by Equation (4). Some other function expressions, for example, the following equation (14) can be used to calculate the importance coefficient K(i). In other words, if the target object data Detect is equal to or larger than a predetermined threshold Th_Detect, flag data is set. The flag data gives instruction to perform the process using a predetermined maximum value K_max as the importance coefficient K(i). In this case, the importance coefficient K(i) is set to the predetermined maximum value K_max. On the other hand, if the target object data Detect is smaller than Th_Detect, K(i) is calculated based on the inter-image variation amount Diff.

$$K(i) = \begin{cases} flag(K\_\max) & :(Detect \geq Th\_Detect) \\ coeff1 \times Diff & :(Detect < Th\_Detect) \end{cases} \quad\quad (14)$$

It has been mentioned in the above-described embodiment that the importance coefficient K(i) is calculated using both the inter-image variation amount between the in-vivo images I(i) and I(i+1) and the target object region extracted from the in-vivo image I(i); however, it is allowable to calculate the importance coefficient K(i) using either one of the inter-image variation amount and the target object region.

Moreover, the cases of displaying the time-series in-vivo images that are taken by the capsule endoscope 10 has been mentioned in the above-described embodiments; however, the embodiment is not limiting. The invention is similarly applicable to other cases for observing other types of time-series images, as far as an important image (or scene) in time-series images is to be searched, and enables efficient confirmation of the contents of the time-series images. For example, the invention can be applied for checking, by human eyes, contents of time-series images taken by a surveillance camera, etc., or for displaying, at a slow speed, a scene in which an important person or object is present in moving images.

INDUSTRIAL APPLICABILITY

As set forth hereinabove, an image display apparatus according to the present invention is suitable for sequentially displaying time-series images.

**Claims**

1. An image display apparatus that displays time-series images, the image display apparatus comprising:

a display-condition receiving unit that receives at least an input that specifies a minimum display rate as a display condition for displaying each image that makes up time-series images;

an importance calculating unit that calculates an importance coefficient that represents the degree of importance of each of the images;

a display-time calculating unit that calculates a display time for each of the images using the display condition that is received by the display-condition receiving unit and the importance coefficient that is calculated by the importance calculating unit; and

a display control unit that performs control so that the images are displayed on a display unit sequentially in a temporal order for the respective display times that are calculated by the display-time calculating unit.

2. The image display apparatus according to claim 1, wherein
the display-condition receiving unit further receives an input that specifies a total display time required for displaying all the time-series images as the display condition, and
the display-time calculating unit calculates, taking the total display time that is received by the display-condition receiving unit into consideration, the display time of each of the images.

3. The image display apparatus according to claim 1 or claim 2, wherein the importance calculating unit includes an inter-image-variation-amount calculating unit that calculates, for each of the images, an inter-image variation amount between images near each other in the temporal order, and the importance calculating unit calculates the importance coefficient of each of the images using the inter-image variation amount that is calculated by the inter-image-variation-amount calculating unit.

4. The image display apparatus according to claim 3, wherein the inter-image-variation-amount calculating unit calculates the inter-image variation amount using at least one of a degree of similarity between images, an optical flow, and a change in an amount of statistics.

5. The image display apparatus according to claim 1, wherein the importance calculating unit includes a target-object extracting unit that extracts a target object region from each of the images, and the importance calculating unit calculates the importance coefficient of each of the images using a result of extraction of the target object region by the target-object extracting unit.

6. The image display apparatus according to claim 1, wherein the display-time calculating unit calculates, if the importance coefficient of an image that is calculated by the importance calculating unit is larger than a predetermined threshold, the display time for the image using the minimum display rate.

7. The image display apparatus according to claim 1, wherein the display-time calculating unit calculates the display time for each of the images using a non-linear function that associates the importance coefficient with the display time.

8. The image display apparatus according to any of claims 1 to 7, wherein
the display-condition receiving unit further receives an input that specifies, as the display condition, a time-series range of target images that are selected to be displayed from the images,
the display control unit performs display control of the images, among the images in the time-series images, in the time-series range that is received by the display-condition receiving unit.

9. An image display program that causes a computer, which includes a display unit on which time-series images are to be displayed, to execute:

a display-condition receiving step of receiving at least an input that specifies a minimum display rate as a display condition for displaying each image that makes up time-series images;

an importance calculating step of calculating an importance coefficient that represents a degree of importance of each of the images;

a display-time calculating step of calculating a display time for each of the images using the display condition that is received at the display-condition receiving step and the importance coefficient that is calculated at the importance calculating step; and

a display control step of performing control so that the images are displayed on the display unit sequentially in a temporal order for the respective display times that are calculated at the display-time calculating step.

# FIG.1

# FIG.2

# FIG.3

PORTABLE RECORDING MEDIUM — 50

70

IMAGE OBTAINING UNIT — 710

INPUT UNIT — 720

CALCULATING UNIT — 750

IMPORTANCE-COEFFICIENT CALCULATING UNIT — 751

INTER-IMAGE VARIATION AMOUNT CALCULATING UNIT — 751a

TARGET-OBJECT EXTRACTING UNIT — 751b

CONTROL UNIT — 760

IMAGE-DISPLAY-CONDITION SETTING UNIT — 761

IMAGE-DISPLAY CONTROL UNIT — 763

STORAGE UNIT — 740

DISPLAY-CONDITION SETTING DATA — 741

IMPORTANCE-COEFFICIENT DATA — 743

DISPLAY-TIME DATA — 745

IMAGE DISPLAY PROGRAM — 747

DISPLAY-TIME CALCULATING UNIT — 753

DISPLAY UNIT — 730

EP 2 157 790 A1

# FIG.4

START

DISPLAY-CONDITION SETTING PROCESS
(SET MINIMUM DISPLAY RATE R_min,
TOTAL DISPLAY TIME T_all,
TIME-SERIES RANGE i_start-to-i_end
OF IN-VIVO IMAGES
TO BE DISPLAYED AS DISPLAY CONDITIONS) — S10

i=i_start — S20

OBTAIN IN-VIVO IMAGES I(i) AND I(i+1) — S30

IMPORTANCE-COEFFICIENT
CALCULATING PROCESS — S40

i=i+1 — S50

S60

i≦i_end? — YES

NO

DISPLAY-TIME CALCULATING PROCESS — S70

DISPLAY IN-VIVO IMAGES SEQUENTIALLY
FOR RESPECTIVE DISPLAY TIMES — S80

END

# FIG.5

W20

MINIMUM
DISPLAY RATE:     I21

B21    [  10  ]  fps

TIME-SERIES     I25     I27
RANGE:     [ 15000 ] TO [ 45000 ]

B23

TOTAL DISPLAY TIME:

[ 40:00 ]  min

I23

# FIG.6

IMPORTANCE-
COEFFICIENT
CALCULATING
PROCESS

CALCULATE INTER-IMAGE VARIATION
AMOUNT BETWEEN IN-VIVO IMAGES
I(i) AND I(i+1) — S401

EXTRACT TARGET OBJECT REGION
FROM IN-VIVO IMAGE I(i) — S403

CALCULATE IMPORTANCE COEFFICIENT
K(i) OF IN-VIVO IMAGE I(i)
USING INTER-IMAGE VARIATION AMOUNT
AND TARGET OBJECT REGION — S405

STORE CALCULATED IMPORTANCE
COEFFICIENT K(i) — S407

RETURN

# FIG.7

DISPLAY-TIME
CALCULATING
PROCESS

↓

CALCULATE MINIMUM VALUE IN
DISPLAY TIME T_min, MAXIMUM VALUE
IN DISPLAY TIME T_max,
AND NORMALIZED IMPORTANCE
COEFFICIENT K'(i) (i=i_start TO i_end) — S701

↓

INITIALIZE DISPLAY-TIME
CALCULATING PARAMETER Param — S703

↓

CALCULATE DISPLAY TIME T(i)
OF EACH IN-VIVO IMAGE I(i)
(i=i_start TO i_end) — S705

↓

CALCULATE DIFFERENCE VALUE E
BETWEEN SUM OF DISPLAY TIMES T(i)
AND TOTAL DISPLAY TIME T_all THAT IS
SPECIFIED AS DISPLAY CONDITION — S707

↓

S709
$|E| \leq$ REFERENTIAL DIFFERENCE VALUE? — NO → 

YES ↓   S711

STORE CALCULATED DISPLAY TIME T(i)
OF EACH IN-VIVO IMAGE I(i)

↓

RETURN

S713
CHANGE DISPLAY-TIME
CALCULATING
PARAMETER Param

# FIG.8

EP 2 157 790 A1

# FIG.9

A30

22

**INTERNATIONAL SEARCH REPORT**

International application No.

PCT/JP2007/062540

A. CLASSIFICATION OF SUBJECT MATTER
*H04N5/93*(2006.01)i, *A61B1/00*(2006.01)i, *H04N5/66*(2006.01)i, *H04N5/915*
(2006.01)i, *H04N7/18*(2006.01)i

According to International Patent Classification (IPC) or to both national classification and IPC

B. FIELDS SEARCHED

Minimum documentation searched (classification system followed by classification symbols)
H04N5/93, A61B1/00, H04N5/66, H04N5/915, H04N7/18

Documentation searched other than minimum documentation to the extent that such documents are included in the fields searched
Jitsuyo Shinan Koho        1922-1996   Jitsuyo Shinan Toroku Koho   1996-2007
Kokai Jitsuyo Shinan Koho   1971-2007   Toroku Jitsuyo Shinan Koho   1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2005-252372 A  (NTT DoCoMo Tokai, Inc.), 15 September, 2005 (15.09.05), Full text; all drawings (Family: none) | 1-9 |
| A | JP 2005-193066 A  (Olympus Corp.), 21 July, 2005 (21.07.05), Full text; all drawings & US 2002/0198439 A1   & US 2005/0250991 A1 | 1-9 |
| A | JP 2004-247817 A  (Matsushita Electric Industrial Co., Ltd.), 02 September, 2004 (02.09.04), Full text; all drawings (Family: none) | 1-9 |

☒  Further documents are listed in the continuation of Box C.          ☐   See patent family annex.

* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search    21 September, 2007 (21.09.07) | Date of mailing of the international search report    02 October, 2007 (02.10.07) |
|---|---|
| Name and mailing address of the ISA/    Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2007/062540 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 8-084319 A  (Canon Inc.),<br>26 March, 1996 (26.03.96),<br>Full text; all drawings<br>(Family: none) | 1-9 |
| E,A | JP 2007-243699 A  (Hitachi, Ltd.),<br>20 September, 2007 (20.09.07),<br>Full text; all drawings<br>(Family: none) | 1-9 |

Form PCT/ISA/210 (continuation of second sheet) (April 2005)

## REFERENCES CITED IN THE DESCRIPTION

**Patent documents cited in the description**

- JP 2980387 B
- JP 2005192880 A